# EUROPEAN PATENT APPLICATION

(11) **EP 1 588 697 A1**
(43) Date of publication of application: **26.10.2005**
(21) Application number: 04009081.3
(22) Date of filing: 16.04.2004
(51) Int. Cl.: A61K 9/06

(54) **Emulsion gel for topical application of pharmaceuticals**

(71) Applicant: Bauer, Kurt H., Prof. Dr., D-79112 Freiburg (DE)
(72) Inventor: Bauer, Kurt H., Prof. Dr., D-79112 Freiburg (DE)
(74) Representative: Becker, Konrad

(57) **Abstract**

The invention relates to a novel emulsion gel ointment for the topical application of pharmaceutically active ingredients. This emulsion gel ointment comprises a hydrogel formed from the aqueous phase and a hydrophilic gel structure former of the type of acrylic acid polymer or copolymer or highly viscous cellulose ether, hydrophilic oxyethylene and oxypropylene block copolymers as emulsifiers, dermatologically well-tolerated natural plant oils or related synthetic or semi-synthetic compounds and phospholipids or other absorption enhancers and optionally liquid waxes as the lipophilic phase, and a pharmaceutically active ingredient. The invention further relates to a method of manufacture of such a formulation.

## Description

### Field of the Invention

The invention relates to a novel emulsion gel ointment for the topical application of pharmaceutically active ingredients.

### Background Art

The therapeutic use of topically administrable pharmaceutical preparations is frequently indicated in those cases where oral or another form of parenteral administration leads to intolerability, risks or harmful side effects or where an undesired biotransformation of the active ingredient occurs. Application to the skin may, therefore, be advantageous if, for example, there is to be a continuous release of the active ingredient component, if active ingredients are intended to act locally or act systemically with the gastro-intestinal tract being by-passed, or if active ingredients having a short biological half-life are to be used.

The possibilities of using customary topically administrable pharmaceutical preparations are limited, for example, by inadequate solubility of the active ingredient or by the inability of such formulations to channel the active ingredient through the skin barrier and thereby enable a systemic action.

Of the various kinds of medicinal formulations that are applied to the skin there may be mentioned, for example, suspensions, solutions, foams and emulsions, such as water-in-oil (W/O) and oil-in-water (O/W) emulsions, and also gels. The nomenclature of "emulsions" is very often used inconsistently. A good summary of the characteristics of emulsions may be found in B.S. Herzog et al., Pharm. Ind. 60, 8, 713 (1998).

A topically administrable pharmaceutical composition in the form of an oil-in-water emulsion gel containing a non-steroidal, anti-inflammatory active compound is described in US Patent 4,917,886.

### Summary of the Invention

The invention relates to a novel emulsion gel ointment for the topical application of pharmaceutically active ingredients. This emulsion gel ointment comprises a hydrogel formed from the aqueous phase and a hydrophilic gel structure former of the type of acrylic acid polymer or copolymer or highly viscous cellulose ether, hydrophilic oxyethylene and oxypropylene block copolymers as emulsifiers, dermatologically well-tolerated natural plant oils or related synthetic or semi-synthetic compounds and phospholipids or other absorption enhancers and optionally liquid waxes as the lipophilic phase, and a pharmaceutically active ingredient. The invention further relates to a method of manufacture of such a formulation.

### Detailed Description of the Invention

This invention relates to a new formulation for a specially designed ointment, and a method of manufacture of such a formulation.

The formulation according to the invention is a hydrogel, into which a lipophilic phase is introduced and emulsified. The lipophilic phase considered consists of dermatologically well-tolerated plant oils, for example natural triglycerides or related synthetic or semi-synthetic compounds, and phospholipids, for example lecithins, or azones, e.g. laurocapram, or dimethylsulfoxide as absorption enhancers, and optionally liquid waxes, e.g. esters of lower alcohols with long-chain fatty acids, as spreading agents, and optionally aroma oils and/or perfumes and/or other customary adjuvants.

The hydrogel is formed from an aqueous phase comprising a gel structure former of the type of acrylic acid polymer or copolymer or highly viscous cellulose ether, and optionally other water-soluble components, in particular low-molecular alcohols, and the active ingredient.

As an emulsifier solid hydrophilic oxyethylene and oxypropylene block copolymers are introduced. These emulsifiers according to the invention are not haemolytically active. It is a particular characteristic of the present invention that other emulsifiers, in particular widely used low-molecular weight emulsifiers, are excluded in order to guarantee a very good physiological tolerance. An *in vitro* test for quick evaluation of the haemolytic activity of surfactants (emulsifiers) based on a physiological approach is known from R. Stenz and K.H. Bauer, Pharmazie 51(5), 283-287 (1996).

The preparations according to the invention may be regarded technologically as emulsion gels, cream gels or gel creams, and may be designated as such. Such an emulsion gel ointment may be used for the manufacture of topically active semi-solid medicaments, whereby suitable topically applicable pharmaceutically active ingredients are added.

A further characteristic of such an ointment according to the invention and the medicaments produced therewith is that the aqueous phase is considerably larger in volume than the lipophilic phase. Because of the larger volume of the aqueous phase and the type of emulsifiers used these novel emulsion gels are oil-in-water (O/W) emulsions.

Preferred emulsifiers are hydrophilic block copolymers of oxyethylenes and oxypropylenes, having hydrophilic polyhydroxyethylene groups and hydrophobic polyhydroxypropylene groups, for example polyoxyethylenepolyoxypropylene polymers, especially having a molecular weight of from approximately 1000 to approximately 11000, so-called poloxamers, for example Pluronics®, for example Pluronic®F68, preferably those showing relatively high HLB values (Hydrophilic Lipophilic Balance) between 18 and 30 and molecular weights above 5000. Preferred pharmaceutical formulations contain poloxamers from approximately 0.5 to approximately 20% by weight of emulsifier, in particular 1 to 10% per weight.

In an emulsion gel of the invention the aqueous phase is thickened to a hydrogel using a hydrophilic gel structure former creating a matrix in which the water of the formulation is stored. Hydrophilic gel structure former are for example polyacrylic acids or polymethacrylic acids, in particular polyacrylic acids, for example carbomers, especially having a molecular weight of from approximately 80'000 to approximately 1 million, or salts thereof, such as Rohagit S®, Eudispert®, or Carbopol® (high molecular weight homo- and copolymers of acrylic acid crosslinked with a polyalkenyl polyether), or highly viscous cellulose ethers, for example hydroxyethylcellulose (HEC), hydroxypropylcellulose (HPC) or methlyhydroxypropylcellulose (MHPC). The preferred percent by weight range of the gel structure former or mixture of gel structure formers is from approximately 0.1 to approximately 5% per weight, preferably 0.4 to 3% per weight. Preferred as hydrophilic gel structure formers are carbomers, e.g. Carbopol®.

The emulsion gels of the invention contain a hydrophilic gel structure former which allows sterilisation by heat without damaging the final emulsion structure.

The aqueous phase may further contain alcohols, in particular low molecular weight alcohols, for example ethanol or isopropanol. Such an alcohol gives a cooling effect on application of the emulsion gel to the skin. If an alcohol is added to the ointment, its percent per weight is preferably up to approximately 20%, in particular 1 to 10%.

If necessary the aqueous phase contains bases for neutralizing acidic groups, i.e. groups that yield protons. Suitable bases, for example, are those that result in the salts of active ingredients described below. Especially preferred as bases are sodium or potassium hydroxide or carbonate, ammonia, and organic amines, for example methylglucamine, lysine, or glucosamine. In addition to the acidic active ingredients, especially gel structure formers having acidic groups are also neutralized. The addition of bases serves especially to adjust the pH value. Consequently, the addition of base may be essential.

The main component of the lipophilic phase is a dermatologically well-tolerated plant oil. Such oils are triglycerides, in particular fatty acid esters of glycerine, it being possible for various fatty acid constituents to occur within the glyceride, however, for the glyceride to be a liquid (an oil), it usually contains an increased content of unsaturated fatty acids. Such oils are e.g. natural plant oils, for example groundnut oil (arachis oil), olive oil, castor oil, sesame oil, soybean oil, and the like. As an alternative to natural plant oils, semi-synthetic and completely synthetic glycerides come into consideration, for example caprylic/capric acid triglyceride, for example Miglyol®812 or Syndermin®GTC. However, natural plant oils are preferred. The preferred percent by weight range of the lipophilic phase oil is from approximately 1 to approximately 20% per weight, preferably 2 to 10% per weight.

Further components of the lipophilic phase are phospholipids, in particular lecithin, or also azones, such as laurocapram (1-dodecylazacycoheptan-2-one), or dimethylsulfoxide, which are absorption enhancers and also show emulsifying properties, but are not haemolytically active. Phospholipids are especially phosphoglycerides, preferably phosphatidyl cholines which are produced by esterification of glycerol-3-phosphoric acid with a saturated and an unsaturated fatty acid, the phosphoric acid residue being for its part esterified by choline (also called lecithins). For example, egg lecithin or soya lecithin are used. The per cent by weight range of the preferred phospholipid lecithin is from approximately 1 to approximately 10% per weight.

Other optional components of the lipophilic phase are liquid waxes with spreading properties, for example isopropyl myristate, isopropyl stearate, ethyl oleate or decyl oleate such as Cetiol®V.

If one-step heat sterilization is important it is preferable to replace the heat-sensitive phospholipids by heat-stable dimethylsulfoxide or laurocapram as listed above. Otherwise heat sterilization is performed at temperatures around 120° Centigrade before adding phospholipids under sterile conditions.

Active ingredients are especially those for systemic treatment that are to be applied to the intact skin, are to enter the skin layers, penetrate these and primarily pass into the circulation of the vascular system of the corium and the subcutis and possibly of the subcutaneous tissue lying beneath the latter and also of the muscle region. Based on the well-tolerable and excellently spreading ingredients the new emulsion gel is also usable for the topical treatment of injured skin surfaces.

Such active ingredients may be any pharmaceuticals suitable and medicinally reasonable for transdermal (topical) application, and may, for example, belong to the classes of analgesics, anti-inflammatory agents, antihistamines, antimycotics, muscle relaxants, vitamins (e.g. vitamin A acid and derivatives, cyanocobalamin) and combinations thereof. The amount of active ingredient in the emulsion gel ointment of the invention is, for example, approximately 0.5 to approximately 30%, preferably between 1 and 20%.

Adjuvants may be added to the ointments, which are standard adjuvants in the pharmacy. Such non-essential constituents of the emulsion gel ointment according to the invention are, if desired, chemical stabilisers, moisture-retaining agents, and/or perfumes, always taking care not to use haemolytically active components.

As chemical stabilisers there come into consideration, for example, anti-oxidants which prevent the oxidative decomposition of active ingredients and adjuncts. Suitable for this purpose are, for example, ascorbyl palmitate or tocopherols (vitamin E). Additional protection against heavy metal anions, for example Cu²⁺ ions, is effected by the addition of complex formers, such as citric acid.

The conditions that must be met by suitable moisture-retaining agents are a high affinity for water, it being necessary that the moisture range be narrow, a high viscosity and good tolerability, for example, as in polyhydric alcohols having at least two hydroxy functions, such as butanediols, glycerol, ethylene glycol or propylene glycol.

Perfumes considered are those generally used in ointments, preferably volatile aroma oils generally recognized as safe.

The emulsion gel of the invention differs from cream gels wherein the aqueous phase is liquid and the lipophilic phase thickened by the addition of fatty alcohols or related waxy ointment components. Both formulations, emulsion gels and cream gels, are in fact emulsions of the O/W type, but they differ substantially in the consistency of their hydrophilic and lipophilic phase. This is of decisive importance for the biopharmaceutical property of the pharmaceutically active ingredient in these ointments, since the mobility and the transfer from ointment to the skin is dependent on the type of phase the medicament is in, the lipophilicity or hydrophilicity of the active ingredient, and the viscosity (liquid or solid, respectively) of the corresponding phase.

The different viscosity properties of the lipophilic and hydrophilic phase are important for influencing the transfer and the speed of transfer of an active ingredient to the skin. The transfer of the active ingredient is determined by the ointment properties, but also whether the active ingredient is predominantly hydrophilic or lipophilic, and the actual distribution between these two phases.

The emulsion gels of the invention are free of the usual low-molecular synthetic emulsifiers. They only comprise natural emulsifiers or polymeric, non-haematolytically active emulsifiers. This represents an important difference to the ointments of the prior art.

The emulsion gels of the invention support the spreading of the drug over the skin surface, but first of all the penetration of the active ingredient into or even through the skin. On application to the skin the aqueous phase enters partially into the skin and makes it more permeable by swelling the skin. The active ingredient partially penetrates the skin, and the residual water and other volatile solvents, for example ethanol or isopropanol, evaporate creating a cool sensation. A thin liquid film consisting of emulsifier(s) and lipids remains on the skin surface. This film further contains active ingredient and the gel-forming compound in dispersed form. The type of dispersion of the active ingredient is decisive for the bioavailability of the medicament.

The active ingredient dissolves und diffuses into the skin at a rate dependent on its properties and on the properties of the remaining film containing lipids and emulsifier(s).

The advantages of this novel formulation reside in its particular composition, for example, in the favourable cosmetic properties, in its properties which allow heat sterilisation, in a distinctly readier solubility of active hydrophilic ingredients and the associated higher effective active ingredient concentration, in the absence of haemolytically active low-molecular weight emulsifiers, and also in a considerably improved chemical stability of the active ingredient in comparison with conventional topical formulations.

The novel ointments are, for example, composed of (percentage given in % per weight):
a) active ingredient in (partially) water soluble form 0.5 - 30%; preferably 1.0 - 20%;
b) lipid phase or lipophilic phase:
   natural plant oil (fatty acid triglyceride) 1.0 - 20%, preferably 2.0 -10%;
   lecithins (phospholipids, absorption enhancers) 1.0 - 10%, preferably 2.0 - 5.0%;
   volatile oils (aroma oils, perfumes) 0.05 - 2.0%;
c) poloxamers (polymeric O/W emulsifier, MW>5000) 0.5 - 20%, preferably 1.0 -10%;
d) aqueous phase:
   deionized or distilled water 60 - 90%, preferably 70 - 85%;
   isopropanol and/or ethanol 0 - 20%, preferably 1.0 - 10%;
   polyacrylic acid (gel structure former, e.g. Carbopol®) 0.1 - 5,0%, preferably 0.4 - 3.0%;
   equivalent amount of base for neutralizing the gel structure former.

The process for the manufacture of the new ointment according to the invention is characterised in that a gel is formed by dispersing the gel structure former in a portion of the water, and the lipophilic phase formed by mixing the lipid constituents, and the active ingredient is added and, if desired, the non-essential constituents are incorporated.

In particular, the manufacture of the new ointment is performed according to standard procedures as known for hydrogels, in the following way:
a) The gel structure former in a small amount of water and/or optionally other hydrophilic solvents, e.g. ethanol, is stirred and dispersed, avoiding lump formation. The polymeric emulsifier (e.g. poloxamer) is dissolved in a further amount of water, under slight heating. The aqueous phases are combined, and further diluted step by step with stirring. Finally the active ingredient is added and mixed with the dispersion.
b) Using standard procedures known in the art, phospholipids or other absorption enhancers (e.g. laurocapram or dimethylsulfoxide) and optionally liquid waxes are dissolved in dermatologically well-tolerated plant oils.
c) Both hydrophilic and lipophilic preparations are finally mixed and emulsified.
d) At the end an approx. 10% aqueous alkaline solution (containing suitable inorganic or organic base as described above) is added to neutralize the polyacrylic acid and potential acidic active ingredients (e.g. diclofenac acid, ibuprofen acid, indomethacin and the like). The required amount for neutralization has to be calculated. On neutralization the mixture starts to solidify and takes its final consistency. It is homogenized with suitable stirring.

However, the order of mixing the gel, active ingredient solution, lipophilic phase and neutralizing agent is not essential in the manufacture of the formulation. The non-essential constituents, such as anti-oxidants or perfumes, may be incorporated into the emulsion gel at any stage, for example into the appropriate aqueous or lipophilic phase before mixing, or also at the end before or after adding the neutralizing base.

For the manufacture of the novel ointment the active ingredients may be used in particular form, e.g. as optical isomers, or particular crystal modifications, and may further be used in pre-treated form, e.g. as a salt with predetermined solubility in water, particular corn size, or encapsulated in a solid solvent. With these pre-treatments a particular rate of diffusion of the active ingredient into the skin may be reached, e.g. a desired controlled penetration or diffusion rate for the desired activity. The diffusion rate is dependent on several parameters, for example on hydrophilicity or lipophilicity of the active ingredient (distribution coefficient) and the particular ointment composition, the phase distribution and the consistency and viscosity of the phases.

If heat sterilization is desired the whole emulsion gel is heated to 120° Centigrade. If the emulsion gel contains heat-sensitive ingredients, only the hydrogel base is heated, and the heat-sensitive components (e.g. the active ingredient and phospholipids) added after reasonable cooling, for example to 50° Centigrade, under aseptic conditions. The ease of heat sterilisation of the emulsion gel ointment of the invention is of high importance given the rigid quality requirements and limits of germ numbers in ointments.

The following Examples illustrate the invention described above but they are not intended to limit the scope thereof in any way. Temperatures are given in degrees Centigrade.

### Example 1:

I) 0,8 kg Carbopol® 934 (polyacrylic acid) are uniformly moistened with 5.0 kg isopropanol and stirred. 44.5 kg water are added in small portions and uniformly stirred avoiding lump formation. After the formation of a homogenous dispersion, 1.0 kg 10% aqueous potassium hydroxide is added for neutralization, and uniformly distributed.
II) 5.0 kg poloxamer 188 (Pluronic® F68) are dissolved in 35.0 kg water with slight heating, and 3.2 kg of diclofenac lysine salt added.
III) 2.5 kg pure lecithin are dissolved in 2.5 kg olive oil under slight heating.
IV) Solution II and dispersion I are mixed together. Solution III is added in small portions, and emulsified by stirring.
V) 0.5 kg volatile melissa oil is added as a perfume, and the whole mixture homogenized.

### Example 2:

I) 1.5 kg Carbopol® 840 NF (polyacrylic acid) are carefully and slowly added to 100.0 kg purified water with stirring until a homogenous, lump-free dispersion is obtained. The residual amount of water (60.5 kg) is added thereafter.
II) 4.0 kg diclofenac acid, 10.0 kg ethanol, 10.0 kg poloxamer 407 (Pluronic® F 127) and 10.0 kg 50% solution of phospholipid in sesame oil (Phosal® SA 50) are mixed together and dissolved, if required by slight heating.
III) Solution II is added in small portions to dispersion I, and emulsified.
IV) 3.0 kg 10% aqueous sodium hydroxide are added to emulsion III in order to neutralize Carbopol® und diclofenac acid, and homogenized by stirring.
V) 0.5 kg fir needle oil and 0.5 kg dwarf pine oil are added as perfume, and the whole mixture homogenized.

### Example 3:

I) 0.75 kg Carbopol® 934 P NF (polyacrylic acid) are carefully added in small portions to 77.25 kg purified water with stirring until a homogenous, lump-free dispersion is obtained.
II) 5.0 kg ibuprofen acid, 5.0 kg ethanol, 5.0 kg Pluronic® F 38, 2.5 kg lecithin and 2.5 kg soybean oil are mixed together and dissolved, if required by slight heating.
III) Solution II is added in small portions to dispersion I, and emulsified.
IV) 1.5 kg 10% aqueous ammonia are added to emulsion III in order to neutralize Carbopol® und ibuprofen acid, and homogenized by stirring.
V) 0.5 kg of an aroma oil mixture is added as perfume, and the whole mixture homogenized.

### Example 4:

I) 0.7 kg Carbopol® 934 (polyacrylic acid, carbomer) are uniformly moistened with 5.0 kg water, and stirred. 42.8 kg water are added in small portions, and stirred to give a lump-free dispersion.
II) 5.0 kg poloxamer 407 (Pluronic® F 127) are dissolved in 27.5 kg water with slight heating.
III) Solution II and dispersion I are mixed together, then 1.0 kg 10% aqueous potassium hydroxide is added for neutralization purposes, and distributed evenly.
IV) The finished hydrogel III is sterilized by heating up to 120°C for 10 minutes, and then slowly cooled down.
V) By aseptic handling 2,5 kg pure lecithin are mixed with 2.5 kg isopropanol, and the mixture dissolved in 2.5 kg castor oil, and then 5,0 kg flufenamin-meglumin salt added and dissolved.
VI) Solution V is added after cooling down under 50°C in small portions to the dispersion III and emulsified.
VII) 0.5 kg of an aroma oil mixture is added as perfume, and the whole mixture homogenized, also under aseptic conditions.

### Example 5:

I) 0,4 kg Carbopol® 980 (polyacrylic acid) are uniformly moistened with 2.5 kg isopropanol and homogeneously kneaded. 26.6 kg water are added in small portions and uniformly stirred avoiding lump formation. After the formation of a homogenous dispersion, 0.5 kg 10% aqueous potassium hydroxide are added for neutralization, and uniformly distributed.
II) 5.0 kg poloxamer 188 (Pluronic® F68) are dissolved in 2.5 kg isopropanol and 25.0 kg water with slight heating, and 1.0 kg of indomethacin added.
III) 1.0 kg hydroxyethylcellulose (Natrosol® HHR) is dispersed in 30.0 kg hot water and subsequently cooled.
IV) 2.5 kg dimethylsulfoxide is mixed with 2.5 kg olive oil.
V) Solution II and the dispersions I and III are mixed together. Then the solution IV is added in small portions, and emulsified by stirring.
V) 0.5 kg volatile pine tree oil is added as a perfume, and the whole mixture homogenized.

### Example 6:

I) 3.0 kg hydroxypropylcellulose (Klucel® HF) are moistened homogeneously with 2.5 kg propylene glycol. 47.0 kg water are added in small portions and uniformly stirred avoiding lump formation.
II) 5.0 kg poloxamer 407 (Pluronic® F127) are dissolved in 2.5 kg propylene glycol and 35.0 kg water with slight heating.
III) 2.5 kg isopropyl myristate are mixed with 2.5 kg arachis oil, and finally 10,000 I.E. retinal acetate are dissolved.
IV) Solution II and dispersion I are mixed together. Solution III is added in small portions, and emulsified by stirring, and the whole mixture homogenized.

## Claims

1. Emulsion gel ointment for the topical application of a pharmaceutically active ingredient comprising
a hydrogel formed from the aqueous phase and a hydrophilic gel structure former of the type of acrylic acid polymer or copolymer or highly viscous cellulose ether, hydrophilic oxyethylene and oxypropylene block copolymers as emulsifiers, dermatologically well-tolerated natural plant oils or related synthetic or semi-synthetic compounds and phospholipids or other absorption enhancers and optionally liquid waxes as the lipophilic phase, and a pharmaceutically active ingredient.

2. Emulsion gel ointment according to claim 1 comprising
a hydrogel formed from the aqueous phase and a hydrophilic acrylic acid polymer or copolymer gel structure former, hydrophilic oxyethylene and oxypropylene block copolymers as emulsifiers, dermatologically well-tolerated natural plant oils and phospholipids as the lipophilic phase, and a pharmaceutically active ingredient.

3. Emulsion gel ointment according to claim 1 comprising
a hydrogel formed from the aqueous phase and a hydrophilic acrylic acid polymer or copolymer gel structure former, hydrophilic oxyethylene and oxypropylene block copolymers as emulsifiers, dermatologically well-tolerated natural plant oils and liquid waxes as the lipophilic phase, and a pharmaceutically active ingredient.

4. Emulsion gel ointment according to claim 1 or 2 comprising
a) active ingredient in water soluble or partially water soluble form;
b) lipid or lipophilic phase comprising dermatologically well-tolerated plant oil, phospholipids, and optionally aroma oils and/or perfumes;
c) poloxamers;
d) aqueous phase comprising water and an acrylic acid polymer or copolymer.

5. Emulsion gel ointment according to claim 1, 2 or 4 comprising
a) 0.5 - 30% (per weight) of an active ingredient in water soluble or partially water soluble form;
b) lipid phase or lipophilic phase comprising 1.0 - 20% (per weight) of a dermatologically well-tolerated plant oil, 1.0 - 10% (per weight) lecithin, and 0.05 - 2.0% (per weight) aroma oils and/or perfumes;
c) 0.5 - 20% (per weight) poloxamers;
d) aqueous phase comprising 60 - 90% (per weight) of water, optionally up to 20% (per weight) isopropanol or ethanol, 0.1 - 5.0% (per weight) of an acrylic acid polymer copolymer, and an equivalent amount of base for neutralizing the emulsion gel ointment.

6. Emulsion gel ointment according to claim 1, 2 or 4 comprising
a) 1.0 - 20% (per weight) of an active ingredient in water soluble or partially water soluble form;
b) lipid phase or lipophilic phase comprising 2.0 -10% (per weight) of a natural plant oil, 2.0 - 5.0% (per weight) lecithin, and 0.05 - 2.0% (per weight) aroma oils and/or perfumes;
c) 1.0 - 10% (per weight) poloxamers;
d) aqueous phase comprising 60 - 90% (per weight) of water, optionally up to 20% (per weight) isopropanol or ethanol, 0.4 - 3.0% (per weight) of an acrylic acid polymer or copolymer, and an equivalent amount of base for neutralizing the emulsion gel ointment.

7. Emulsion gel ointment according to claim 1, 2 or 4 comprising
a) 1.0 - 20% (per weight) of an active ingredient in water soluble or partially water soluble form;
b) lipid phase or lipophilic phase comprising 2.0 - 10% (per weight) of a natural plant oil, 2.0 - 5.0% (per weight) lecithin, and 0.05 - 2.0% (per weight) aroma oils;
c) 1.0 -10% (per weight) poloxamers;
d) aqueous phase comprising 60 - 90% (per weight) of water, 1.0 - 10% (per weight) isopropanol or ethanol, 0.4 - 3.0% (per weight) of a carbomer, and an equivalent amount of base for neutralizing the emulsion gel ointment.

8. Emulsion gel ointment according to anyone of claims 1 to 7 wherein the active ingredient is vitamin A acid, a vitamin A derivative, or cyanocobalamin.

9. A method of manufacture of an emulsion gel ointment according to anyone of claims 1 to 8 wherein
a) the gel structure former is dissolved in water, the emulsifier is dissolved in a further amount of water, and the aqueous phases and the active ingredient are combined;
b) the phospholipids or other absorption enhancers and optionally liquid waxes are dissolved in dermatologically well-tolerated plant oils;
c) both hydrophilic and lipophilic preparations are mixed and emulsified; and
d) the mixture is neutralized and homogenized.

10. A method of manufacture according to claim 9 further comprising heat sterilization.
